# EUROPEAN PATENT SPECIFICATION

(11) **EP 4 301 246 B1**
(45) Date of publication and mention of the grant of the patent: **31.12.2025**
(21) Application number: 22763681.8
(22) Date of filing: 16.02.2022
(51) Int. Cl.: A61B 17/11, A61M 1/00

(54) **A SYSTEM FOR NEGATIVE PRESSURE ANASTOMOSIS**
SYSTEM FÜR UNTERDRUCKANASTOMOSE
SYSTÈME D'ANASTOMOSE À PRESSION NÉGATIVE

(30) Priority: 02.03.2021 SE 2150233
(43) Date of publication of application: 10.01.2024
(73) Proprietor: Carponovum AB, 302 41 Halmstad (SE)
(72) Inventor: GRÖNBERG, Anders, 302 70 Halmstad (SE)
(74) Representative: Ström & Gulliksson AB
(86) International application number: PCT/SE2022/050171
(87) International publication number: WO 2022/186747

(56) References cited:
- EP-A1- 2 389 961
- EP-A2- 2 857 051
- CN-U- 207 941 058
- US-A1- 2009 105 734
- US-A1- 2009 105 734
- US-A1- 2010 168 720
- US-A1- 2013 079 890
- US-A1- 2014 031 773
- US-A1- 2017 281 301

## Description

### Technical field

The present disclosure relates to anastomosis of a living tissue, and more particularly to a system for anastomosis of tubular structures.

### Background

Colorectal cancer is the third most frequent type of cancer in the world having an occurrence of about 1 million new cases every year. The incidents of cancer are considerably more frequent in the industrial part of the world.

During removal of a tumour from living tissue, for example a tubular structure such as an intestine, the affected part of the living tissue is removed by cutting the living tissue at a suitable distance on each side. Depending on where and how the tumour was located, there may be a need to reconnect the living tissue. In the case of a tubular structure, this process is known as anastomosis.

A major issue regarding anastomosis healing is the blood circulation of the anastomosis during the healing process. Despite substantial development of surgical techniques during the last decades, morbidity and mortality after resections in the gastrointestinal tract, e.g., due to anastomotic leakage, remain as serious problems. Another problem arising from stapling of anastomosis is anastomotic stenosis. The critical area for healing, i.e., the contact area between the two ends of the hollow structure to be connected, has to be liquid proof, and the cross section of the lumen should be as wide and flexible as the original lumen.

There has therefore been a need in the technical field to develop assemblies overcoming these disadvantages. One such assembly is disclosed in WO 2007/122223, wherein a device comprising interlocking members for use in achieving anastomosis of tubular organs is disclosed. The device comprises two rigid parts, onto which two elastic rings are arranged. Two intestine ends are respectively secured between each rigid part and corresponding elastic part, after which the rigid parts are interconnected via a connection member. Another such assembly is disclosed in WO2014/031065, wherein a similar device for use in the side wall of an intestine is disclosed.

EP 2857051 A2 discloses another device for the treatment of internal injury to the gastrointestinal tract.

EP 2389961 A1 discloses a medical device i.e. medical low pressure device, for controlling wound healing process of patient, has electronic control device producing control signal such that pulsations of pulses is synchronized with alternating pressure pulses.

US 2014/031773 A1 discloses a medical device includes a longitudinal axis member, a suction member that is provided at a distal end of the longitudinal axis member, has a contact surface capable of coming into close contact with an inner wall of a bursiform cavity when inserted into the bursiform cavity, and has a plurality of communication passages that are open to the contact surface and communicate with a lumen, a suction source provided at a proximal end of the longitudinal axis member so as to be connected to the lumen, and a closing member that is provided at an axial end of the suction member, comes into contact with the opening, and airtightly seals the opening.

It is however difficult to control and monitor the healing process with such devices, as they are arranged in the intestine until they are naturally passed from the body the body sometime after surgery.

### Summary

Accordingly, the present disclosure preferably seeks to mitigate, alleviate or eliminate one or more of the above-identified deficiencies and disadvantages in the art singly or in any combination. This is achieved by a system comprising a pump in fluid communication with a cavity formed between an anastomotic device and a tubular structure. The system can be used to control and monitor the healing process of the anastomosis formed by the device.

The present invention is defined by the features of the independent claim. Further embodiments are provided by the dependent claims.

According to an aspect, there is provided a system for anastomosis of a tubular structure, the system comprising a device configured such that, when the device is arranged in the tubular structure, an anastomosis is obtained in the tubular structure and a cavity is formed between the device and the tubular structure, and a pump in fluid communication with the cavity and configured to temporarily apply a negative pressure to the cavity.

The pump is configured to apply the negative pressure to the cavity periodically. Optionally, the period at which the pump is configured to apply the negative pressure to the cavity is inversely proportional to the heart rate of a patient having the tubular structure. Optionally, the period at which the pump is configured to apply the negative pressure to the cavity is an integer multiple of the time between consecutive heartbeats of a patient having the tubular structure. Optionally, the magnitude of the negative pressure is lower than the magnitude of the diastolic pressure of a patient having the tubular structure. Optionally, the pump is a vacuum pump.

Optionally, the system further comprises at least one catheter in fluid communication with the cavity at a first end, and in fluid communication with the pump at a second end. Optionally, in use, the second end of the at least one catheter is configured to pass through the tubular structure and exit through the rectum of a patient having the tubular structure. Optionally, the at least one catheter comprises at least two catheters. Optionally, a first catheter of the at least two catheters is configured as an inlet to the cavity for a contrast medium, and a second catheter of the at least two catheters is configured as an outlet from the cavity for the contrast medium. Optionally, the at least two catheters comprise four catheters.

Optionally, the system further comprises a pressure sensor configured to measure the pressure in the cavity. Optionally, the pressure sensor is integrated with the pump. Optionally, the system further comprises comprising an absorbent element disposed between the pump and the cavity.

The tubular structure comprises a first part and a second part, and the device is configured such that, when the device is arranged in the tubular structure the anastomosis is obtained at a contact area between the first part of the tubular structure and the second part of the tubular structure, and the cavity is formed between the device, the first part of the tubular structure and the second part of the tubular structure.

The device comprises a first member and a second member of generally hollow open configurations such that, when the device is arranged in the tubular structure, the anastomosis is obtained at a contact area between the first and second members and the cavity is formed between the first and second members and the tubular structure. Optionally, the first member and/or the second member is provided with at least one hole arranged to enable the fluid communication between the pump and the cavity. Each of the first member and the second member comprises a rigid part and an elastic part arranged such that the contact area is between the elastic parts and the cavity is formed between the rigid parts and the tubular structure. Optionally, the elastic parts are ring shaped. Optionally, the elastic parts are made of a polymeric material, such as an elastomer. Optionally, the elastic parts are made of a biocompatible material and/or a biodegradable material. Optionally, each elastic part and corresponding rigid part, in use, form a continuous line of contact in which necrosis is induced in the tubular structure. Optionally, the rigid parts are configured to connect to each other such that a distance is formed between the elastic parts.

According to another aspect, there is provided a method (not forming part of the claimed invention) for anastomosis of a tubular structure, the method comprising arranging a device in a tubular structure such that an anastomosis is obtained in the tubular structure and a cavity is formed between the device and the tubular structure, and applying a negative pressure to the cavity temporarily using a pump in fluid communication with the cavity.

Optionally, the method comprises applying the negative pressure to the cavity periodically. Optionally, the period at which the pump is configured to apply the negative pressure to the cavity is inversely proportional to the heart rate of a patient having the tubular structure. Optionally, the period at which the pump is configured to apply the negative pressure to the cavity is an integer multiple of the time between consecutive heartbeats of a patient having the tubular structure. Optionally, the method comprises applying the negative pressure at a magnitude lower than the magnitude of the diastolic pressure of a patient having the tubular structure. Optionally, the pump is a vacuum pump.

Optionally, the method further comprises arranging at least one catheter between the pump and the cavity, such that a first end of the catheter is in fluid communication with the cavity, and a second end of the catheter is in fluid communication with the pump. Optionally, the method comprises arranging the at least one catheter such that the second end of the at least one catheter passes through the tubular structure and exits through the rectum of a patient having the tubular structure. Optionally, the at least one catheter comprises at least two catheters. Optionally, the method comprises providing a contrast medium to the cavity via a first catheter of the at least two catheters, and removing the contrast medium from the cavity via a second catheter of the at least two catheters. Optionally, the at least two catheters comprise four catheters.

Optionally, the method further comprises measuring the pressure in the cavity. Optionally, the method comprises measuring the pressure in the cavity using a pressure sensor integrated with the pump. Optionally, the method further comprises disposing an absorbent element between the pump and the cavity.

Further objects, features and advantages of the present disclosure will appear from the following detailed description, from the attached drawings as well as from the dependent claims.

### Brief description of the drawings

Exemplary embodiments of the disclosure shall now be described with reference to the drawings in which:
Fig. 1 schematically illustrates a system for anastomosis of living tissue according to one embodiment;
Fig. 2a is a perspective exploded view of an anastomotic device according to one embodiment;

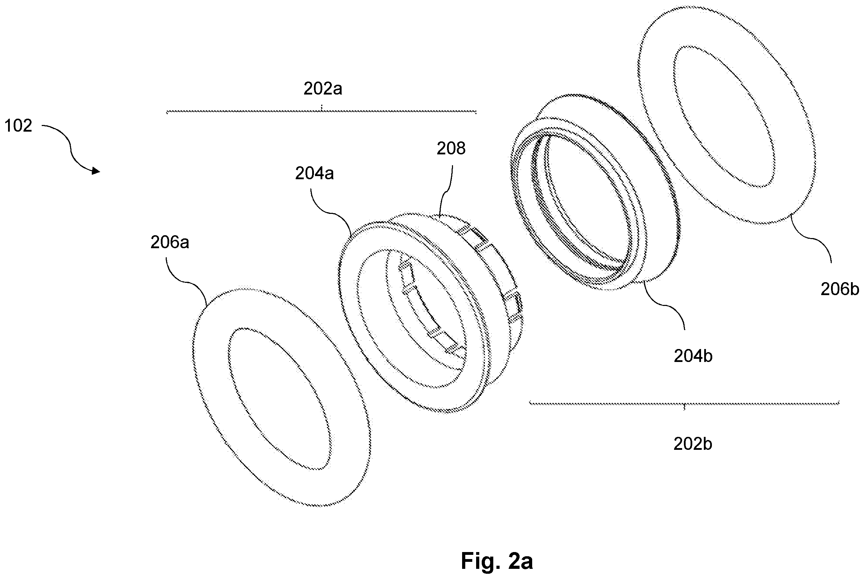

Fig. 2a is a perspective view of a member of the anastomotic device of fig. 2a according to one embodiment;

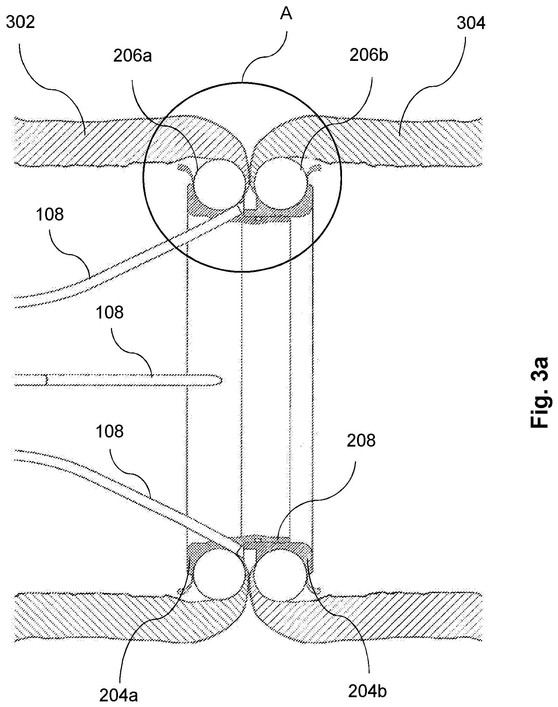

Fig. 3a is a cross sectional view of an anastomotic device arranged in a tubular structure;

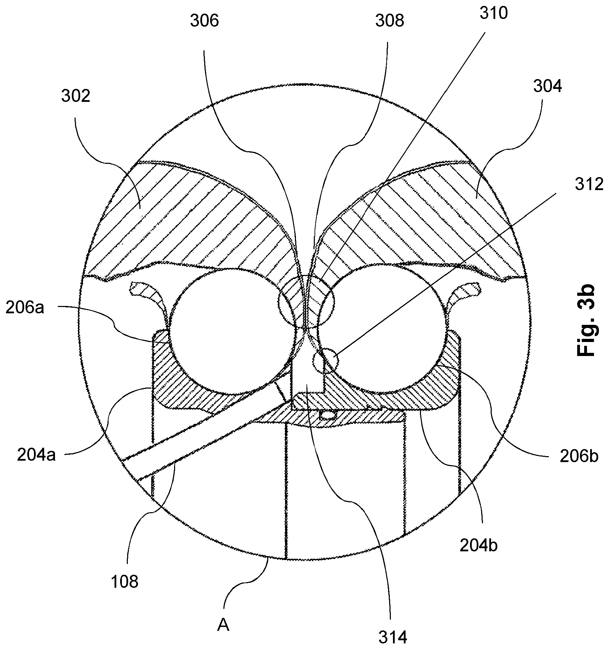

Fig. 3b shows an enlarged view of a section of Fig. 3a; and

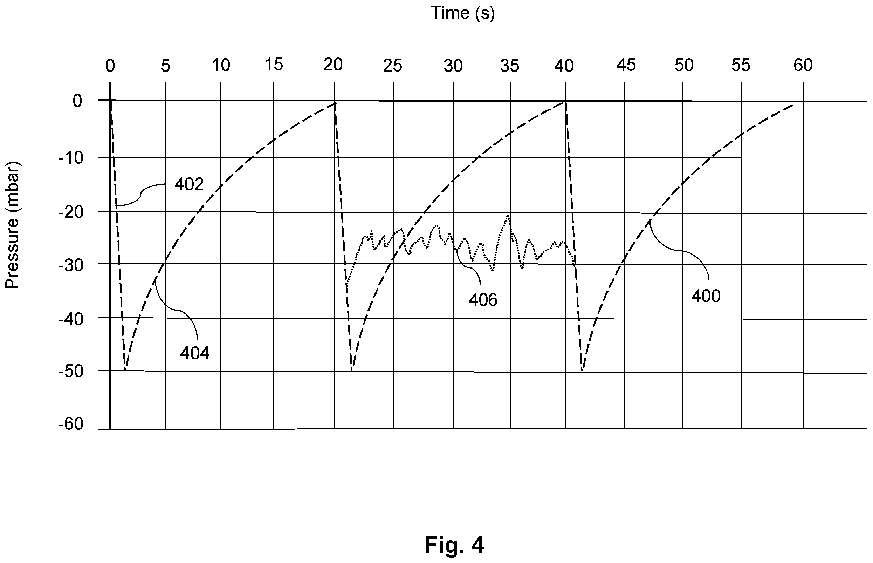

Fig. 4 shows an example readout from a pressure sensor of the system.

Throughout the description and the drawings, like reference numerals refer to like parts.

### Detailed description

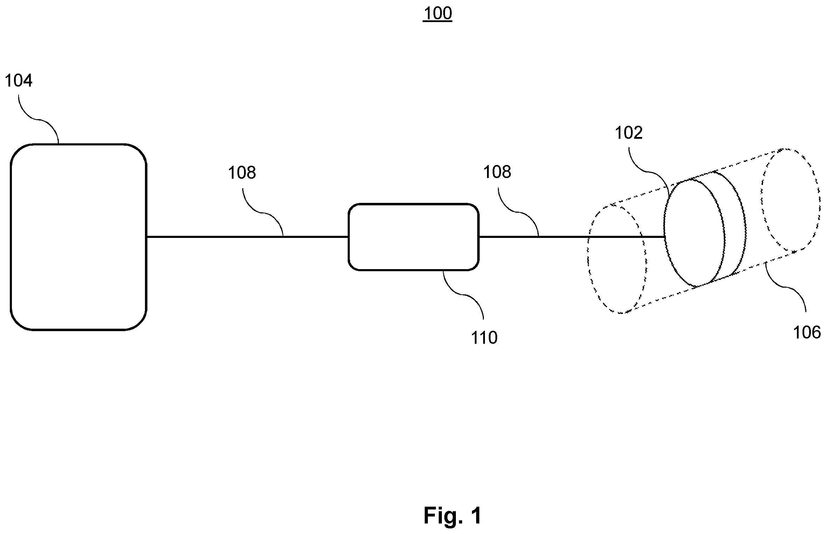

Fig. 1 schematically illustrates a system 100 for anastomosis of living tissue, in particular a tubular structure. The system 100 comprises an anastomotic device 102 and a pump 104. In use, the device 102 is arranged in a tubular structure 106, such as an intestine. As will be discussed below, when the device 102 is arranged in the tubular structure 106, a cavity is formed between the device 102 and the tubular structure 106. The pump 104 is in fluid communication with the cavity, for example via one or more catheters 108. The pump 104 is configured to apply a negative pressure to the cavity. In some embodiments, an absorbent element 110 is disposed between the pump 104 and the device 102 or the cavity. Whilst the system 100 may typically be used for anastomosis of an intestine, it will be appreciated that it may also be implemented in other tubular structures of a body for anastomosis of those structures.

To explain the operation of the system 100 further, an example of the device 102 will be explained in more detail. The device 102 may be an anastomotic device such as those disclosed in WO2007122223 and/or WO2014/031065, however any anastomotic device configured such that an anastomosis is obtained in the tubular structure and a cavity is formed between the device and the tubular structure when the device is arranged in the tubular structure could be used.

An exploded view of an example device 102 is shown in Fig. 2a. The device 102 comprises a first member 202a and a second member 202b that are connectable to each other. The first member 202a comprises a first rigid part 204a and a first elastic part 206a. The second member 202b comprises a second rigid part 204b and a second elastic part 206b. The first and second members 202a, 202b, in particular the rigid parts 204a-b and the elastic parts 206a-b, may have a generally hollow open configuration.

The rigid parts 204a-b are ring shaped, with a side or recess adapted to receive a corresponding elastic part 206a-b. The recess has a shape that is at least partly complementary to the shape of the elastic part 206a-b. The rigid parts 204a-b may be formed of a polymeric material, more specifically a biocompatible material and most specifically a biodegradable material, of a rigidity adequate to stabilize the elastic parts 206a-b.

The elastic parts 206a-b are also ring shaped. The elastic parts 206a-b may be made as a compact body or a tube filled with air, gas or fluid. The elastic parts 206a-b may be made of a polymeric or rubber material, such as an elastomer, of for example 40 to 70 Shore. In some embodiments, the elastic parts 206a-b are made of a biocompatible material and/or a biodegradable material. The elastic parts 206a-b can be only partially elastic. The elasticity is used for squeezing the tubular structure 106 between the elastic parts 206a-b and the rigid parts 204a-b, respectively, with a certain force, as will be explained below. It will be envisaged that other means for performing the same function are also possible. The elastic parts 206a-b have a circular cross section as shown in Figs. 3a-b, but can be of any other shape, e.g. elliptic or oval, or partly rectangular or triangular, hexagonal, octagonal etc., and can be segmented.

To form the first and second members 202a, 202b, a respective elastic part 206a-b is arranged in the recess of the rigid part 204a-b. As such, the elastic parts 206a-b have an inner diameter that is smaller than the smallest outer diameter of the rigid parts 204a-b. The elastic part 206a-b may be attached to the rigid part 204a-b through gluing, over-moulding or co-moulding. The hollow open configuration of the first and second members 202a-b have a circular cross section as shown in Figs. 3a-b, but can be of any other shape, e.g. elliptic or oval, or partly rectangular or triangular, hexagonal, octagonal etc., and can be segmented.

To assemble the device 102, the rigid parts 204a-b of the first and second member 202a-b are configured to connect to each other such that a distance is formed between their respective elastic parts 206a-b. The device 102 may additionally comprise a connection member 208 for joining the first member 202a to the second member 202b. In one embodiment, the connection member 208 is integral with the first rigid part 204a, although it will be appreciated that the connection member 208 could be integral with the second rigid part 204b or could be a separate element. The first member 202a and the second member 202b may be interlockable to each other, as a male-female component, using the connection member 208.

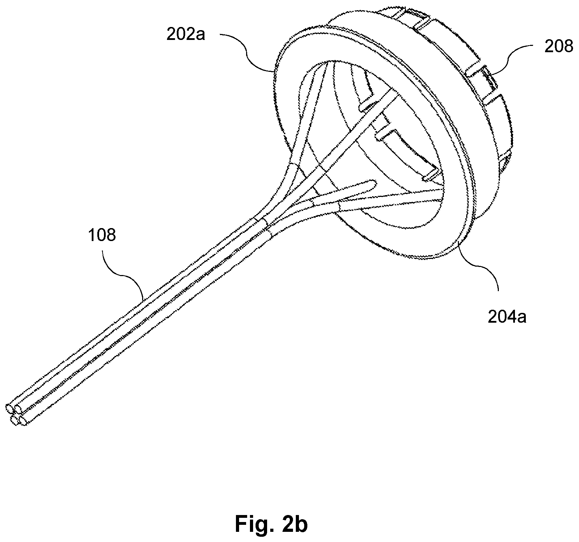

Fig. 2b shows the first member 202a provided with four catheters 108. The catheters 108 may be mounted to respective holes in the first rigid part 204a. The holes may allow the catheters 108 to be in fluid communication with a cavity formed between the device 102 and the tubular structure 106, as will be described in relation to Figs 3a-b. Whilst four catheters 108 are shown in Fig. 2b, any suitable number of catheters 108 may be implemented. In some implementations, a single catheter 108 may be used. In other implementations, a plurality of catheters 108 may be used, for example two, four or any other suitable number. In the case that a plurality of catheters 108 are implemented, the holes may be arranged symmetrically around the annular wall of the first rigid part 204a.

The device is arranged to connect two free ends of a tubular structure 106, or arranged in a side wall of a tubular structure 106. When connecting a free end of a tubular structure 106, the first member 202a is placed in the lumen of the tubular structure 106 such that it is in contact with the inner wall of the tubular structure 106 and is positioned around the incision created during the incising of the wall of the tubular structure 106. After positioning the first member 202a within the tubular structure 106, the intestine end is secured between each rigid part 204a and corresponding elastic part 206a. After positioning the first member 202a within the lumen of the tubular structure 106, the second member 202b is positioned correspondingly the other free end of the tubular structure 106. The first member 202a and the second member 202b may then be connected, for example using the connection member 208. For example, the first member 202a and the second member 202b may be snap fit into cooperation.

Fig. 3a shows a cross sectional view of the device 102 arranged in a tubular structure 106. The tubular structure 106 has a first end 302 and a second end 304, which are to be fused together. In the example described, the device 102 has a first rigid part 204a provided with a number of catheters 108. The first elastic part 206a is arranged inside the tubular structure 106 at the first end 302, and the edge of the first end 302 is folded over the first elastic part 206a. The first rigid part 204a is inserted into the tubular structure 106 from the first end 302, expanding the first elastic part 206a, which is surrounded or wrapped on three sides by the tubular structure 106, until it is snapped around the circumference of the first rigid part 204a. The first elastic part 206a is then locked to the first rigid part 204a in the recess of the first rigid part 204a. Thus, a single layer of the tubular structure 106 is squeezed between the first rigid part 204a and the first elastic part 206a. The operation described is repeated for arranging the second member 202b in the second end 304 of the tubular structure 106. Thus, the first and second ends 302, 304 of the tubular structure 106 are secured in the first and second members 202a-b of the device 102, respectively. The final action for installing the device 102 is to connect the first member 202a and the second member 202b using the connection member 208, which can be performed by a simple press action by hand by an operator.

Fig. 3b shows an enlarged view of the section A of Fig. 3a. The enlarged view shows the compression of the tubular structure 106 between the rigid parts 204a-b and the elastic parts 206a-b. The extremity of the first end 302, when folded and arranged between the first rigid part 204a and the first elastic part 206a, forms a first lip or a contact surface 306 that is essentially circular. Similarly, the second end 304 forms a second lip or contact surface 308. An essentially uniform and equal pressure is exerted on the contact surfaces 306, 308 all over the periphery.

When the device 102 is arranged in the tubular structure 106, a contact area 310 is created between the contact surfaces 306, 308. The contact area is formed between the first and second members 202a-b, specifically between the elastic parts 206a-b. Anastomosis is obtained at the contact area 310. That is to say, tissue regeneration takes place at the contact area 310, which will result in fusing together the two ends 302, 304 of the tubular structure 106.

A point of necrosis 312 (indicted only on the second end 304) is formed at the point where the tubular structure 106 is pressed against the rigid part 204a-b by the pressure from the corresponding elastic part 206a-b. The blood stream or circulation at the ends 302, 304 of the tubular structure 106 is cut off (strangled) and ceases right up to the point of necrosis 312. The point of necrosis 312 extends around the ends 302, 304 of the tubular structure 106 such that it may also be considered as a continuous line of necrosis 312. When the two ends of the tubular structure 106 are fused, i.e. have healed, the tissue separates at the line of necrosis 312 and the device 102 is automatically released from the tubular structure 106. The device 102 may then leave the tubular structure 106, for example following the faecal stream through the rectum. This may occur at different times for different patients, but will typically be between one and three weeks after surgery, for example around 10 to 12 days. In some cases, the device 102 may leave the tubular structure 106 through a stoma opening.

The first and second members 202a-b are dimensioned so that a gap is present between the first and second elastic parts 206a-bwhen the members 202a-b are interlocked without arrangement of the two ends 302, 304 of the tubular structure 106 between them. As shown in Fig. 3b, when the device 102 is arranged in the tubular structure 106, the ends 302, 304 of the tubular structure will swell up and form a closed cavity 314 defined by the compressed tubular structure 106 and the device 102. Specifically, the cavity is formed between the rigid parts 204a-b of the first and second members 202a-b and the two ends 302, 304 of the tubular structure 106. First ends of the catheters 108 are arranged in the holes in the rigid part 204a. The first ends of the catheters 108 open into the cavity 314 and are therefore in fluid communication with the cavity 314. The catheters 108 extend from the device 102, through and out of the tubular structure 106, for example through the rectum. Second ends of the catheters 108 can thus be put into fluid communication with devices external of the body, such as the pump 104, syringes, or other devices used for supervising or controlling the healing of the ends 302, 304 of the tubular structure 106. It is also possible to supply different fluids to the cavity 314 in a continuous or an intermittent flow. The fluid could be grow stimulating substances or a contrast medium, as will be explained below.

The pump 104 can therefore be put in fluid communication with the cavity 314 via the catheters 108. The pressure in the cavity 314 has an equilibrium value, i.e., the undisturbed pressure in the cavity 314. This may be the atmospheric pressure. The pump 104 can be used to apply a negative pressure to the cavity 314, for example by drawing fluid from the cavity 314. The pump 104 may be a suction pump or a vacuum pump. The negative pressure applied by the pump 104 is measured relative to the equilibrium value. The pump 104 is configured to apply the negative pressure to the cavity 314 temporarily. As such, pressure in the cavity 314 may be decreased to a predetermined level by the pump 104, at which point the pump 104 is deactivated (or a valve is closed) and the pressure in the cavity 314 recovers to its equilibrium value. As such, the pressure in the cavity 314 is decreased and increased. This aids the healing process at the contact area 310, as healing is stimulated by mobility in the tissue, which is caused by the changes in pressure. As the negative pressure is only applied for a short time, it is also ensured that blood is regularly supplied to the healing area of the tubular structure 106.

The pump 104 is configured to apply the negative pressure to the cavity 314 periodically. As such, the pressure in the cavity 314 is regularly decreased and increased, which further stimulates mobility in the tissue. The period may be set based on the heart rate of a patient having the tubular structure 106. In particular, as the heart rate of a patient increases, the period at which the negative pressure is applied may decrease. That is to say, the period is inversely proportional to the heart rate. In some embodiments, the period is an integer multiple of the time between consecutive heartbeats of the patient. In some embodiments, the pump 104 may be configured to apply the negative pressure to the cavity 314 repeatedly, but at non-periodic intervals, for example randomly.

The level of the negative pressure applied by the pump 104 may be dependent on a blood pressure of the patient, in particular, the diastolic pressure in the tubular structure 106. If level of the negative pressure applied by the pump 104 is higher than the diastolic pressure in the tubular structure 106, only the structure of the wall of the tubular structure 106 will keep it open. If the tubular structure 106 collapses, flow of blood to the healing area will be reduced, leading to failure of the healing process. To avoid this scenario, the magnitude of the negative pressure applied by the pump 104 should be lower than the magnitude of the diastolic pressure in the tubular structure 106. Typical levels of negative pressure applied by the pump 104 may be between - 40mbar and -80mbar, but may vary from patient to patient.

The pumping regime discussed above may be applied for a sufficient time for the healing process to be successfully initiated. For example, the pumping regime may be applied for up to 48 hours after surgery, which corresponds to the critical healing period for such surgery. In some embodiments, the pumping regime may be applied for more or less than 48 hours.

The pumping regime can be controlled externally, for example by changing the period or the level at which the negative pressure is applied by the pump 104. In some embodiments, the pumping regime can be controlled by a computer or mobile application, as will be discussed below.

In order for the pump 104 to apply the negative pressure to the cavity 314, only a single catheter 108 is required. A single catheter 108 provides fluid communication between the pump 104 and the cavity 314, such that the pump 104 can control to pressure in the cavity 314. In some embodiments, a plurality of catheters 108 are implemented between the pump 104 and the cavity 314. In one embodiment, such as that shown in Fig. 2b, four catheters 108 are implemented between the pump 104 and the cavity 314.

In some embodiments, the absorbent element 110 is disposed between the pump 104 and the cavity 314, for example at a point along the length of the catheters 108. This ensures that any liquids or contaminants that are extracted from the cavity 314 during operation of the pump 104, for example blood or pus, does not enter into the pump 104 and contaminate it or hinder its operation.

To monitor the healing process, a pressure sensor can be implemented to measure the pressure in the cavity. The pressure sensor may be integrated with the pump 104, or in some embodiments may be integrated in the device 102. Fig. 4 shows an example readout 400 from a pressure sensor. The readout 400 shows the pressure relative to the equilibrium pressure value in the cavity 314 over time. As such, the equilibrium pressure value in the cavity 314 is shown as a zero value. In this configuration, the pump 104 implements a pressure of -50mbar to the cavity once every 20s. After the pressure is reduced by the pump 104 at a first stage 402, there is a recovery stage 404 where the pressure returns to its equilibrium value. This process is repeated periodically, as discussed above.

The pressure sensor provides real-time feedback regarding healing of the anastomosis. In this manner, it is possible to supervise the healing process of the two ends 302, 304 of the tubular structure 108 continuously. For example, if the pressure reaches the equilibrium level during the recovery stage 404 as expected, then it can be surmised that the healing process is going as planned. However, if there are any changes from the expected readout, this can indicate problems in the healing process. For example, if the pressure does not decrease to the expected level, or takes a longer time than expected to achieve that level, this may be indicative of a hole in the tubular structure 106 adjacent to the healing area. Fig. 4 also shows a readout 406 where a hole is temporarily formed in the tubular structure 106, but is closed when the negative pressure is next applied by the pump 104.

The readout 400 may be provided on a display, such as a display of the pump 104 or a separate display such as a computer display for review by an operator, such as a doctor or nurse. In some embodiments, the readout 400 or the associated data can be sent to a remote location for review by an operator. The information in the readout 400 could also be sent to a computer or mobile application, for review by an operator. The application could also be used to control the pumping regime, for example by changing the period or the level at which the negative pressure is applied by the pump 104. Such changes could be based on a baseline status of a patient, for example their age and stability, and the monitored values provided by the pressure sensor, to ensure that the optimal period and/or pressure level is applied for that particular patient.

In some implementations, the system 100 may also be used to visualise the anastomosis during surgery and/or healing. To achieve this, a contrast medium may be supplied through the catheters 108 for performing a radiological control of the anastomosis, for example regarding closeness or contrary leakage, which is especially important directly after mounting the device 102 to the tubular structure 106.

To enable this, a first catheter 108 may be configured as an inlet for the contrast medium, and a second catheter 108 configured as an outlet for the contrast medium. As the contrast medium is supplied to and from the cavity 314, a scan can be made to visualise the presence of the contrast medium in the cavity 314. For example, an X-ray scan can be made to show the contrast medium relative to the tubular structure 106. This can indicate if there is a leak in the anastomosis. In another implementation, a certain pressure of the contrast medium can be supplied to one catheter 108, while the other catheters 108 are kept closed. This enables a measure of the pressure in the cavity 314 to be taken in order to detect any leakage. The absorbent element 110 may again be used to prevent any contrast medium from entering into the pump 104 and contaminate it or hinder its operation.

The system 100 disclosed above can be used to control and monitor the healing process of an anastomosis formed in a tubular structure by an anastomotic device. The pump 104 can apply the negative pressure to the cavity 314 periodically, which aids the healing process. The negative pressure may be set at a suitable value to prevent collapse of the tubular structure 106 during healing. This pumping regime may be applied for up to 48 hours after surgery, which corresponds to the critical healing period. The pumping regime can be controlled externally, for example by a computer or mobile application. An absorbent element may be implemented between the pump 104 and the cavity 314 to prevent any liquids or contaminants that are extracted from the cavity 314 from entering the pump 104 and contaminating it or hindering its operation. A pressure sensor can be used to provide real-time feedback regarding healing of the anastomosis. The system 100 may also be used to visualise the anastomosis during surgery and/or healing, for example using a contrast medium, to detect any leakage from the anastomosis.

In the claims, the term "comprises/comprising" does not exclude the presence of other elements or steps. Furthermore, although individually listed, a plurality of means, elements or method steps may be implemented. Additionally, although individual features may be included in different embodiments, these may possibly be combined in other ways, and the inclusion in different embodiments does not imply that a combination of features is not feasible. In addition, singular references do not exclude a plurality. The terms "a", "an" does not preclude a plurality. Reference signs in the claims are provided merely as a clarifying example and shall not be construed as limiting the scope of the claims in any way.

## Claims

1. A system for anastomosis of a tubular structure comprising a first part (302) and a second part (304), the system comprising:
a device (102) comprising a first member (202a) and a second member (202b) that are connectable to each other, wherein the first member (202a) comprises a first rigid part (204a) and a first elastic part (206a) and the second member (202b) comprises a second rigid part (204b) and a second elastic part (206b), wherein the device (102) is configured such that, when the device (102) is arranged in the tubular structure, an anastomosis is obtained at a contact area (310) between the first part (302) of the tubular structure and the second part (304) of the tubular structure and a cavity is formed between the device (102), a first part of the tubular structure and a second part of the tubular structure; and
a pump (104) in fluid communication with the cavity;
**characterised in that**
the pump (104) is configured to periodically apply a negative pressure to the cavity.

2. The system of any preceding claim, further comprising at least one catheter (108) configured to be fluid communication with the cavity at a first end when the device is arranged in the tubular structure, an anastomosis is obtained in the tubular structure and a cavity is formed between the device and the tubular structure, and further being in fluid communication with the pump at a second end.

3. The system of claim 2, wherein the at least one catheter (108) comprises at least two catheters.

4. The system of any preceding claim, further comprising a pressure sensor configured to measure the pressure in the cavity.

## Patentansprüche

1. System zur Anastomose einer röhrenförmigen Struktur, die einen ersten Teil (302) und einen zweiten Teil (304) aufweist, wobei das System Folgendes aufweist:
eine Vorrichtung (102) mit einem ersten Element (202a) und einem zweiten Element (202b), die miteinander verbunden werden können, wobei das erste Element (202a) einen ersten starren Teil (204a) und einen ersten elastischen Teil (206a) aufweist, und das zweite Element (202b) einen zweiten starren Teil (204b) und einen zweiten elastischen Teil (206b) aufweist, wobei die Vorrichtung (102) so konfiguriert ist, dass, wenn die Vorrichtung (102) in der röhrenförmigen Struktur angeordnet ist, an einer Kontaktfläche (310) zwischen dem ersten Teil (302) der röhrenförmigen Struktur und dem zweiten Teil (304) der röhrenförmigen Struktur eine Anastomose erhalten wird, und zwischen der Vorrichtung (102), einem ersten Teil der röhrenförmigen Struktur und einem zweiten Teil der röhrenförmigen Struktur ein Hohlraum gebildet wird; und
eine Pumpe ( 104) in Fluidverbindung mit dem Hohlraum;
**dadurch gekennzeichnet, dass**
die Pumpe (104) konfiguriert ist, um periodisch einen Unterdruck auf den Hohlraum auszuüben.

2. System nach einem vorstehenden Anspruch, das ferner mindestens einen Katheter (108) aufweist, der konfiguriert ist, dass er an einem ersten Ende in Fluidverbindung mit dem Hohlraum steht wenn die Vorrichtung in der röhrenförmigen Struktur angeordnet ist, eine Anastomose in der röhrenförmigen Struktur erhalten wird, und ein Hohlraum zwischen der Vorrichtung und der röhrenförmigen Struktur gebildet wird, und der ferner an einem zweiten Ende in Fluidverbindung mit der Pumpe steht.

3. System nach Anspruch 2, wobei der mindestens eine Katheter (108) mindestens zwei Katheter aufweist.

4. System nach einem vorstehenden Anspruch, ferner aufweisend einen Drucksensor, der konfiguriert ist, um den Druck in dem Hohlraum zu messen.

## Revendications

1. Système d'anastomose d'une structure tubulaire comprenant une première partie (302) et une seconde partie (304), le système comprenant :
un dispositif (102) comprenant un premier élément (202a) et un second élément (202b) qui peuvent être reliés l'un à l'autre, dans lequel le premier élément (202a) comprend une première partie rigide (204a) et une première partie élastique (206a) et le second élément (202b) comprend une seconde partie rigide (204b) et une seconde partie élastique (206b), dans lequel le dispositif (102) est configuré de telle sorte que, lorsque le dispositif (102) est disposé dans la structure tubulaire, une anastomose est obtenue au niveau d'une zone de contact (310) entre la première partie (302) de la structure tubulaire et la seconde partie (304) de la structure tubulaire et une cavité est formée entre le dispositif (102), une première partie de la structure tubulaire et une seconde partie de la structure tubulaire ; et
une pompe (104) en communication fluidique avec la cavité ;
**caractérisé en ce que**
la pompe (104) est configurée pour appliquer périodiquement une pression négative à la cavité.

2. Système selon une quelconque revendication précédente, comprenant en outre au moins un cathéter (108) conçu pour être en communication fluidique avec la cavité à une première extrémité lorsque le dispositif est disposé dans la structure tubulaire, une anastomose est obtenue dans la structure tubulaire et une cavité est formée entre le dispositif et la structure tubulaire, et étant en outre en communication fluidique avec la pompe à une seconde extrémité.

3. Système selon la revendication 2, dans lequel l'au moins un cathéter (108) comprend au moins deux cathéters.

4. Système selon une quelconque revendication précédente, comprenant en outre un capteur de pression configuré pour mesurer la pression dans la cavité.
